# EUROPEAN PATENT APPLICATION

(11) **EP 4 707 800 A1**
(43) Date of publication of application: **11.03.2026**
(21) Application number: 24797430.6
(22) Date of filing: 25.04.2024
(51) Int. Cl.: G01N 33/49, G01N 1/10

(54) **BLOOD ANALYSIS CARTRIDGE ASSEMBLY AND BLOOD ANALYSIS DEVICE COMPRISING SAME**

(30) Priority: 25.04.2023 KR 20230053790
(71) Applicant: I-SENS, INC., Seoul 06646 (KR)
(72) Inventor: CHOI, Wonseok, Seoul 06646 (KR); KONG, Seunghyun, Seoul 06646 (KR); KIM, Phillip, Seoul 06646 (KR)
(74) Representative: HGF
(86) International application number: PCT/KR2024/005611
(87) International publication number: WO 2024/225776

(57) **Abstract**

A cartridge assembly for blood analysis according to one aspect of the present disclosure includes a main cartridge including a main case having a sub-cartridge accommodation groove formed on one surface thereof, a plurality of reagent solution bags disposed inside the main case, a specimen supply part disposed inside the main case, and an opening/closing door which is elastically and rotatably coupled to another surface facing the one surface, a sub-cartridge which is inserted into the sub-cartridge accommodation groove so as to be detachable from the main cartridge and includes a sub-case and a plurality of quality control solution bags disposed inside the sub-case, and a sensor card detachably coupled to the main cartridge.

## Description

### Technical Field

The present disclosure relates to a cartridge assembly for blood analysis and a blood analysis device including the same.

### Background Art

Typically, the concentrations of electrolytes, gases, and metabolites in the blood or hematocrit maintain homeostasis within the human body, and when an imbalance in concentration occurs, it causes various diseases along with symptoms of excess or deficiency in concentration. Therefore, rapid measurement of the concentrations of electrolytes, gases, and metabolites in the blood or hematocrit may help medical professionals to quickly predict functional abnormalities and disease progression based on biological imbalances.

Conventional blood analysis devices are complex and large in structure due to the nature of mechanical devices, and are therefore have the disadvantage of being used only in specific laboratories, limiting their location of use. In addition, conventional blood analysis devices require periodic replacement of various types of reagents and sensors, which is cumbersome in terms of maintenance.

To address these issues, a point-of-care test (POCT) type blood analysis device has been developed that improves mobility and miniaturizes the device to enable testing on-site rather than in a laboratory.

This POCT type blood analysis device has a specimen supply part including a needle to introduce the analysis target specimen to the blood analysis device, and if foreign substance other than the specimen penetrates into specimen supply part, it may affect the sensor card.

This foreign substance intrusion issue may affect test results and even lead to malfunction of the blood analysis device.

Therefore, the inventor of the present disclosure completed the present disclosure after a long period of research and trial and error on a POCT type blood analysis device.

### [Prior-Art Document]

### Korean Patent No. 10-0886914

### Disclosure of the Invention

### Technical Goals

One object of the present disclosure is to provide a cartridge assembly for blood analysis capable of preventing foreign substances from penetrating into a specimen supply part.

Another object of the present disclosure is to provide a cartridge assembly for blood analysis that has substantially no change in outer shape even when a sub-cartridge including a quality control solution is mounted.

Another object of the present disclosure is to provide a cartridge assembly for blood analysis capable of coupling and separating a main cartridge including a reagent solution and a sub-cartridge including a quality control solution relatively easily.

Another object of the present disclosure is to provide a cartridge assembly capable of increasing ease of handling.

Another object of the present disclosure is to provide a cartridge assembly capable of increasing the types and numbers of reagent solutions and quality control solutions disposed within the cartridge assembly.

Another object of the present disclosure is to provide a cartridge assembly capable of preventing the reagent solution of the cartridge assembly from leaking into the interior of a blood analysis device.

Another object of the present disclosure is to provide a blood analysis device that has substantially no change in outer shape even when a sub-cartridge including a quality control solution is mounted.

Another object of the present disclosure is to provide a blood analysis device capable of easily discharging a cartridge assembly.

Meanwhile, other unspecified objects of the present disclosure will be additionally considered within the scope that may be easily inferred from the detailed description below and the effects thereof.

### Technical Solutions

According to an aspect of the present disclosure, there may be provided a blood analysis device including a blood analysis device main body including a frame in which an accommodation space is formed, and a cartridge assembly which is inserted into the accommodation space and includes a main cartridge comprising a main case, a plurality of reagent solution bags disposed inside the main case, and a specimen supply part disposed inside the main case, a sub-cartridge which is detachably inserted into a sub-cartridge accommodation groove formed on one surface of the main cartridge and includes a plurality of quality control solution bags disposed therein, and a sensor card detachably coupled to the main cartridge, wherein the cartridge assembly is inserted into the blood analysis device main body so that one surface of the main case faces the accommodation space of the frame, and the main cartridge further includes an opening/closing door which is elastically and rotatably coupled to another surface of the main case facing the one surface of the main case, and is opened and closed by the specimen supply part.

Here, the blood analysis device main body may further include a driving part coupled to the frame, the specimen supply part may include a first rotating body coupled to the driving part to rotate, a guide having one end part coupled to the first rotating body, a second rotating body coupled to the guide and configured to slide in a slit formed in the guide, and a needle coupled to the second rotating body, and the second rotating body may press to open the opening/closing door when rotating.

**In** addition, the blood analysis device main body may further include a discharge part coupled to the frame and configured to rotate to discharge the cartridge assembly from the accommodation space of the frame.

**In** addition, a handle accommodation groove may be formed on an upper surface of the main cartridge connected to the one surface of the main cartridge, the cartridge assembly may further include a handle having one end part which is elastically and rotatably coupled to the upper surface of the main cartridge, and the handle may be accommodated in the handle accommodation groove by being pressed by the frame when the cartridge assembly is inserted into the accommodation space of the frame.

According to another aspect of the present disclosure, there may be provided a cartridge assembly for blood analysis including a main cartridge including a main case having a sub-cartridge accommodation groove formed on one surface thereof, a plurality of reagent solution bags disposed inside the main case, a specimen supply part disposed inside the main case, and an opening/closing door which is elastically and rotatably coupled to another surface facing the one surface, a sub-cartridge which is inserted into the sub-cartridge accommodation groove so as to be detachable from the main cartridge and includes a sub-case and a plurality of quality control solution bags disposed inside the sub-case, and a sensor card detachably coupled to the main cartridge.

Here, the specimen supply part may include a first rotating body, a guide having one end part coupled to the first rotating body, a second rotating body coupled to the guide and configured to slide in a slit formed in the guide, and a needle coupled to the second rotating body, and the opening/closing door may be opened by being pressed by the second rotating body.

In addition, the main cartridge may further include a handle having one end part rotatably coupled to the main case.

In addition, the handle may be elastically rotated with respect to the main case.

In addition, a handle accommodation groove may be formed on an upper surface of the main case connected to the one surface of the main case, and the handle may be rotated and accommodated in the handle accommodation groove.

In addition, the main cartridge may further include a solution distribution part disposed on the one surface of the main case, and the solution distribution part may have a plurality of inlets connected to two or more of the reagent solution bags and the plurality of quality control solution bags, and a single outlet.

In addition, the plurality of inlets and the outlet of the solution distribution part may be spaced apart from each other in one direction.

In addition, the sub-cartridge may further include an accommodation connector formed with a plurality of accommodation holes connected to the plurality of the quality control solution bags, respectively, and disposed in one region of the sub-case, and the main cartridge may further include an insert connector formed with a plurality of protrusion parts configured to connect the solution distribution part with the plurality of quality control solution bags, respectively, by being inserted into the plurality of accommodation holes, and disposed in one region of the sub-cartridge accommodation groove corresponding to the accommodation connector.

In addition, the main cartridge may further include a sensor card accommodation connector disposed in the main case and connected to the outlet of the solution distribution part, and the sensor card may be at least partially inserted into the sensor card accommodation connector and connected to the outlet of the solution distribution part.

In addition, a valve coupling hole may be formed on the one surface of the main case, the valve coupling hole into which a valve of the reagent solution bag is inserted and coupled, the main cartridge may further include a leakage receiving part formed on a lower side of the valve coupling hole in the one surface of the main case, and the leakage receiving part may include a support plate protruding from the one surface of the main case toward an outer side of the main case, and a partition wall extending upward from one end part of the support plate.

In addition, a leakage movement hole may be formed between the valve coupling hole and the support plate of the leakage receiving part in the one surface of the main case, and a level of an inner bottom surface of the main case may become lower from the one surface side of the main case toward another surface side of the main case, which is an opposite surface of the one surface of the main case.

In addition, a level of a bottom surface of the support plate may become lower from the one end part side of the support plate toward a region of the support plate in contact with the one surface of the main case.

### Advantageous Effects

According to one aspect of the present disclosure, external foreign substances may be prevented from penetrating into a specimen supply part.

According to another aspect of the present disclosure, even if a sub-cartridge including a quality control solution is mounted on a main cartridge including a reagent solution, the outer shape of the main cartridge may not substantially change.

According to another aspect of the present disclosure, the main cartridge including the reagent solution and the sub-cartridge including the quality control solution may be relatively easily coupled and separated from each other.

According to another aspect of the present disclosure, the handleability and transportability of the cartridge assembly may be increased.

According to another aspect of the present disclosure, the types and numbers of reagent solutions and quality control solutions disposed within the cartridge assembly may be increased.

According to another aspect of the present disclosure, the solution of the cartridge assembly may be prevented from leaking into the interior of the blood analysis device main body.

According to another aspect of the present disclosure, even if the sub-cartridge including the quality control solution is mounted on the blood analysis device main body, the outer shape of the blood analysis device main body may not substantially change.

According to another aspect of the present disclosure, the cartridge assembly may be easily discharged.

It is to be noted that, even effects not explicitly mentioned herein, the effects described below in the specification and their potential effects, which are expected by the technical features of the present disclosure, are to be regarded as set forth in this specification.

### Brief Description of Drawings

FIG. 1 is a diagram schematically illustrating a front surface side of a cartridge assembly for blood analysis according to an embodiment of the present disclosure.
FIG. 2 is a diagram schematically illustrating a rear surface side of a cartridge assembly for blood analysis according to an embodiment of the present disclosure.
FIGS. 3 and 4 are diagrams each schematically illustrating a separated view of a main cartridge and a sub-cartridge of a cartridge assembly for blood analysis according to an embodiment of the present disclosure.
FIG. 5 is a diagram schematically illustrating a coupled cross-section of an insert connector of a main cartridge and an accommodation connector of a sub-cartridge in a cartridge assembly for blood analysis according to an embodiment of the present disclosure.
FIG. 6 is a diagram schematically illustrating a separated view of a main cartridge and a sensor card of a cartridge assembly for blood analysis according to an embodiment of the present disclosure.
FIG. 7 is a diagram schematically illustrating a coupled view of an accommodation connector of a main cartridge and a sensor card in a cartridge assembly for blood analysis according to an embodiment of the present disclosure.
FIG. 8 is a diagram illustrating a part of a cross-section along line I-I' of FIG. 2.
FIG. 9 is a diagram schematically illustrating a blood analysis device according to an embodiment of the present disclosure.
FIG. 10 is a diagram schematically illustrating a blood analysis device main body applied to a blood analysis device according to an embodiment of the present disclosure.
FIG. 11 is a diagram schematically illustrating an exploded view of some components of a blood analysis device main body applied to a blood analysis device according to an embodiment of the present disclosure.
FIG. 12 is a diagram schematically illustrating a cartridge assembly pipeline opening/closing structure of a blood analysis device according to an embodiment of the present disclosure.
FIG. 13 is a diagram schematically illustrating an operating structure of a specimen supply part and an opening/closing door of a blood analysis device according to an embodiment of the present disclosure.
FIG. 14 is a diagram schematically illustrating a cartridge assembly discharge structure of a blood analysis device according to an embodiment of the present disclosure.
FIG. 15 is a diagram schematically illustrating a connection relationship between components of a blood analysis device according to an embodiment of the present disclosure.

It is to be understood that the attached drawings are provided for reference only to help understand the technical idea of the present disclosure, and the scope of the rights of the present disclosure is not limited thereby.

### Best Mode for Carrying Out the Invention

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

In addition, throughout the specification, "on" means located above or below the target part, and does not necessarily mean located on the upper side based on the direction of gravity. In addition, the term "coupling" is used as a concept that includes not only the case where each component is physically in direct contact with each other, but also the case where another component is interposed between each component, and each component is in contact with the other component.

Since the size and thickness of each component shown in the drawings are arbitrarily shown for the convenience of description, the present disclosure is not necessarily limited to what is shown.

In the drawing, a first direction may be defined as a direction in which a cartridge assembly is inserted into a blood analysis device main body. In addition, a direction opposite to the first direction may be defined as at least one of a direction in which a sub-cartridge is inserted into a main cartridge, a direction in which a sensor card is inserted into the main cartridge, and a direction in which the cartridge assembly is discharged from the blood analysis device main body.

Hereinafter, a cartridge assembly for blood analysis and a blood analysis device including the same according to an embodiment of the present disclosure will be described in detail with reference to the accompanying drawings, and in describing with reference to the accompanying drawings, identical or corresponding components will be assigned the same drawing numbers and overlapping descriptions thereof will be omitted.

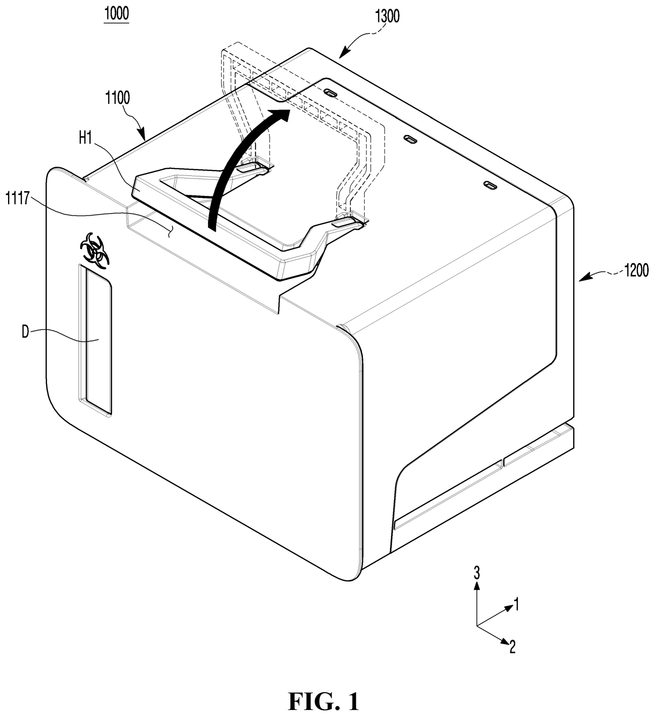

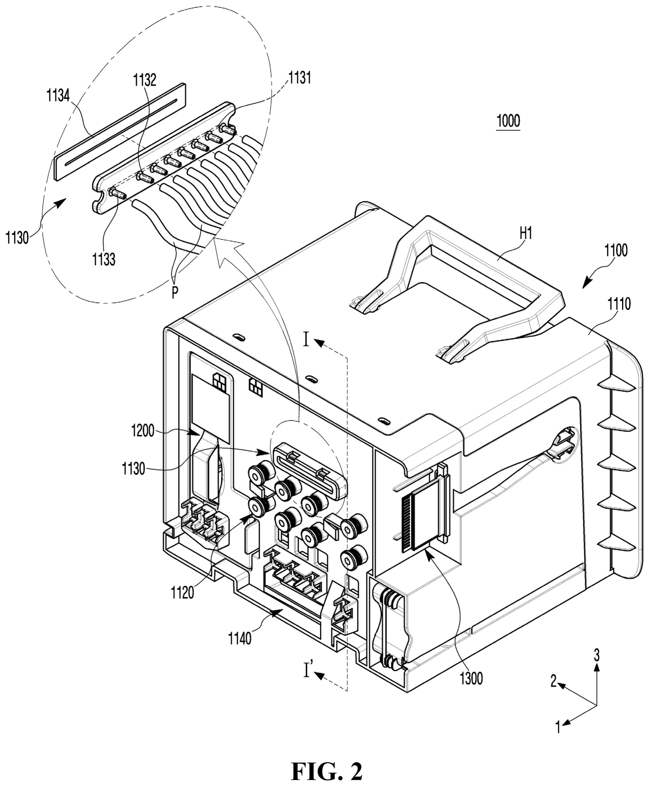

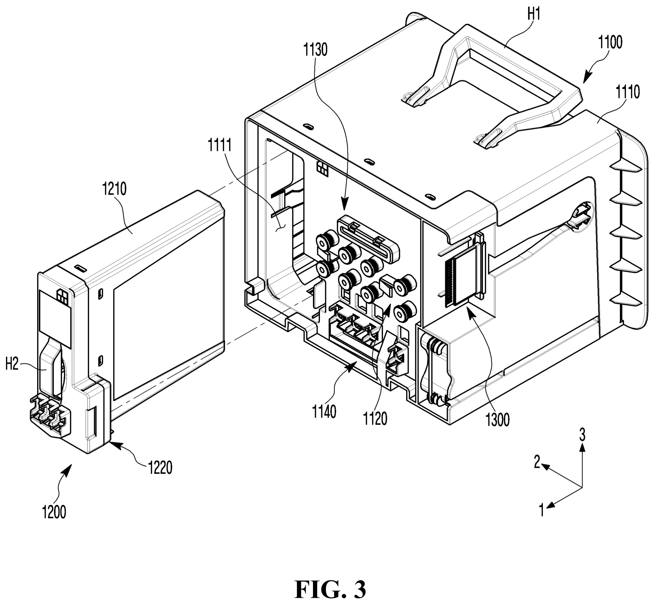

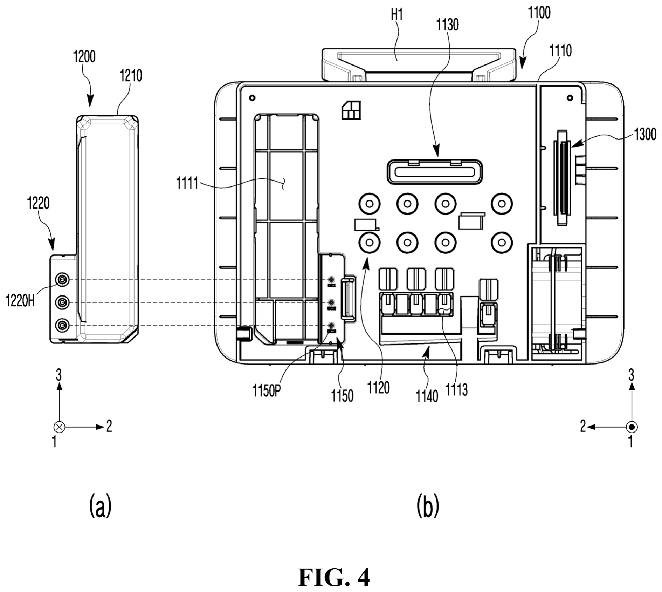

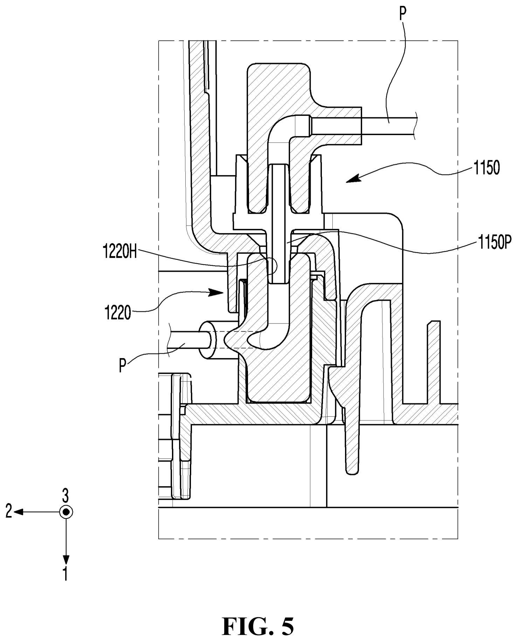

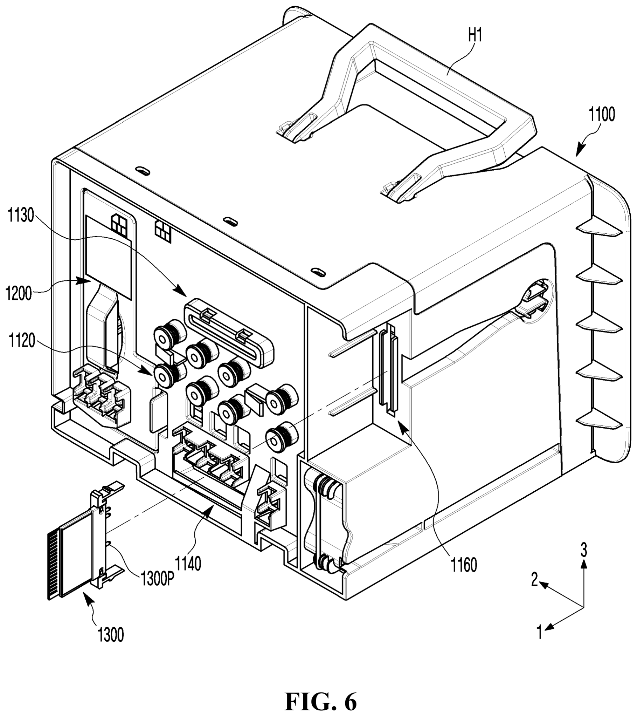

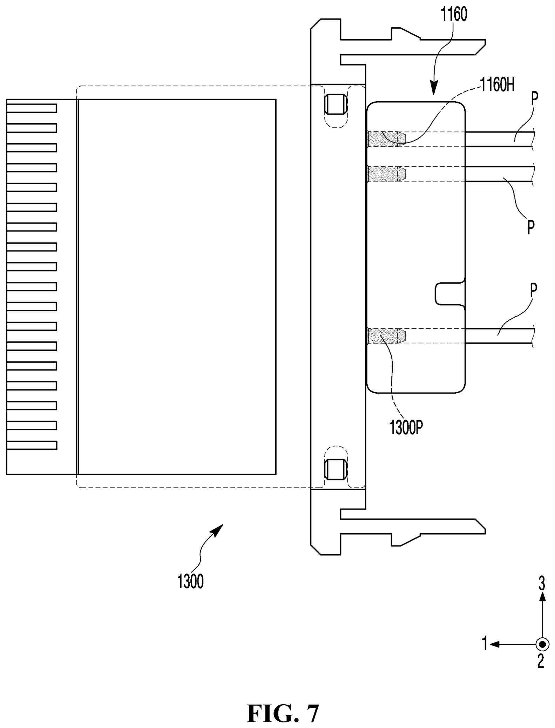

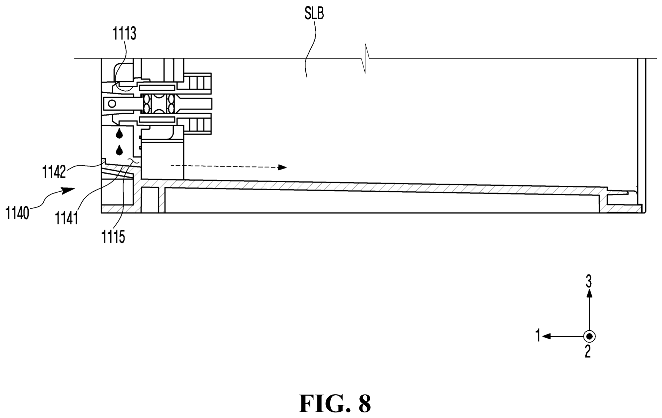

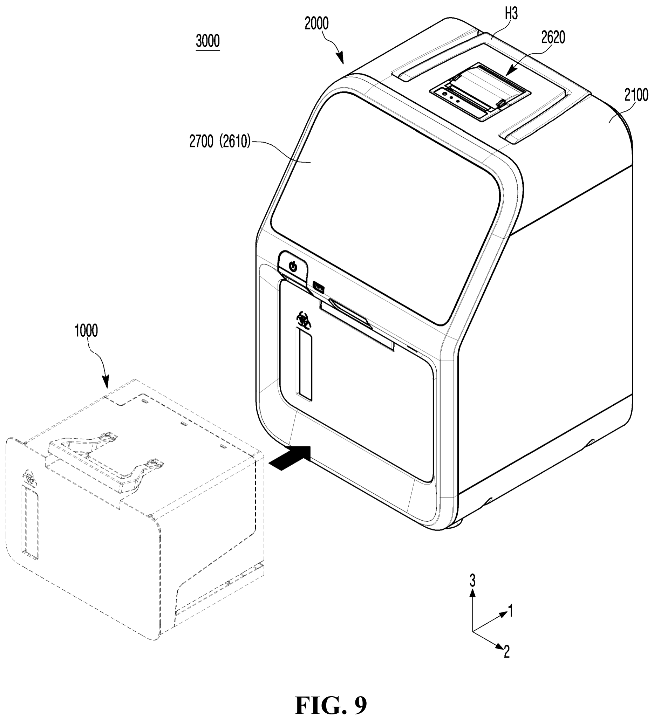

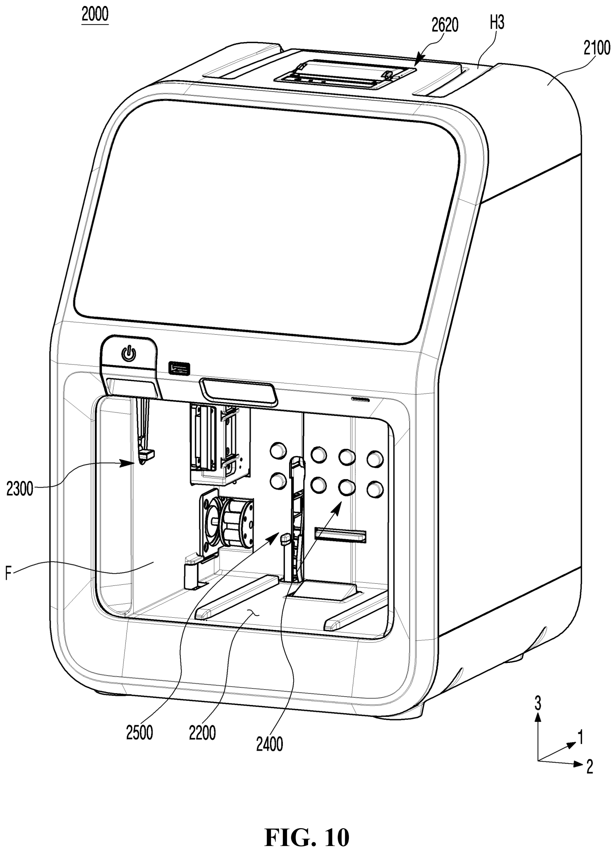

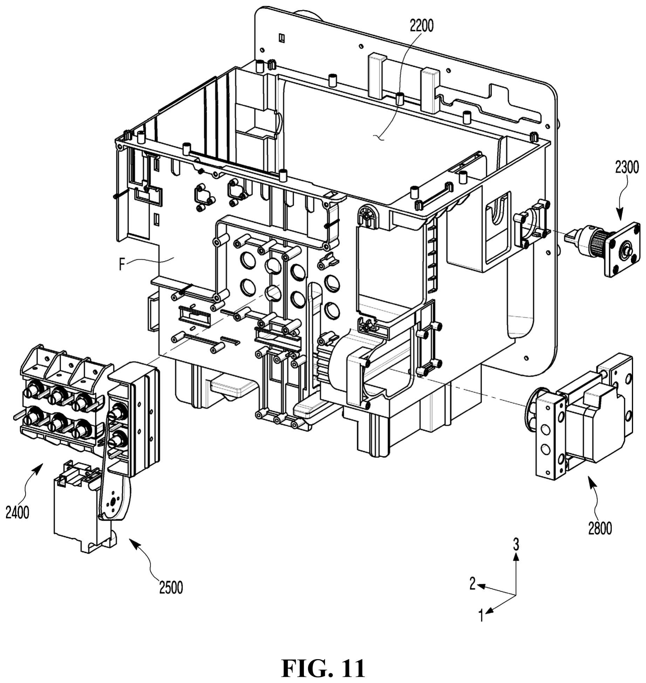

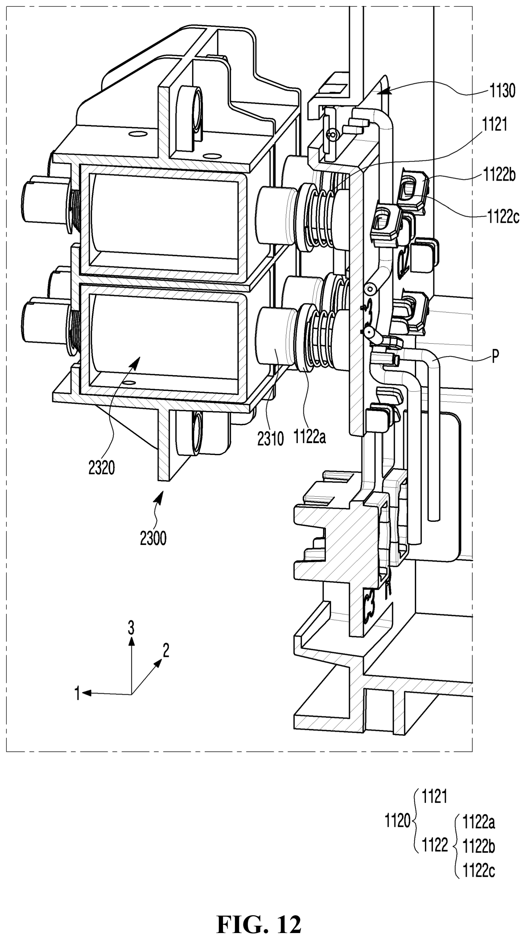

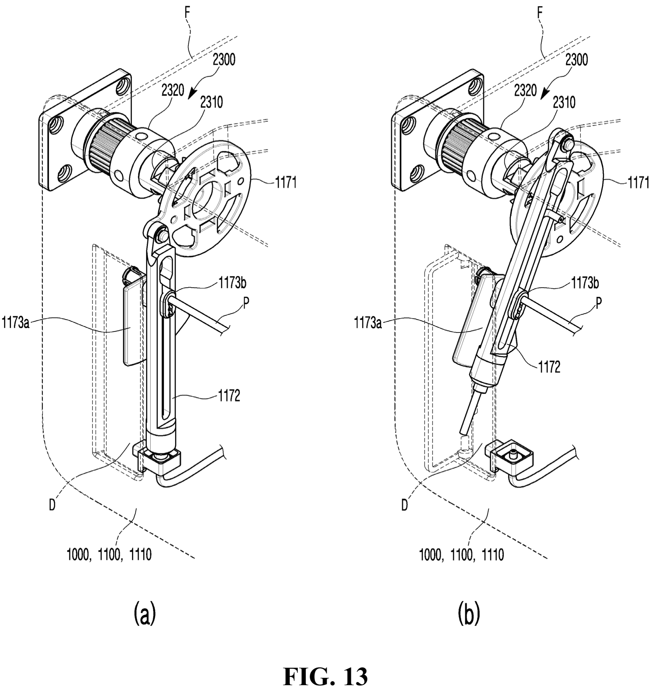

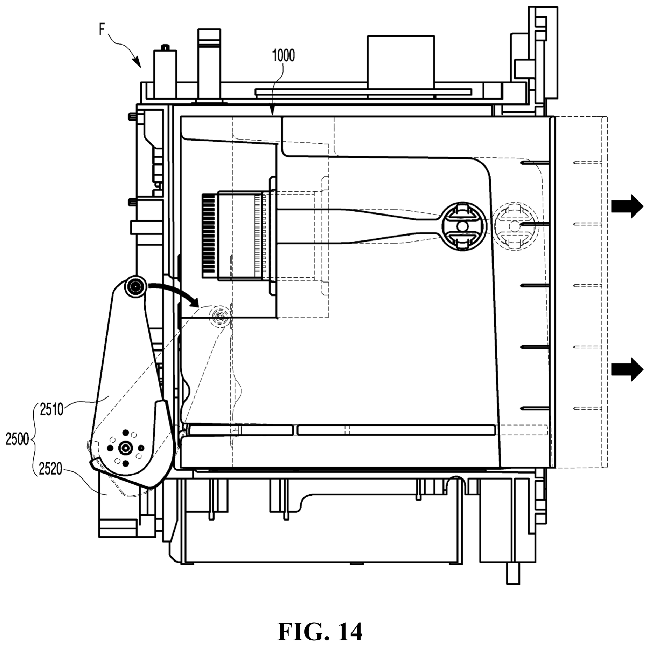

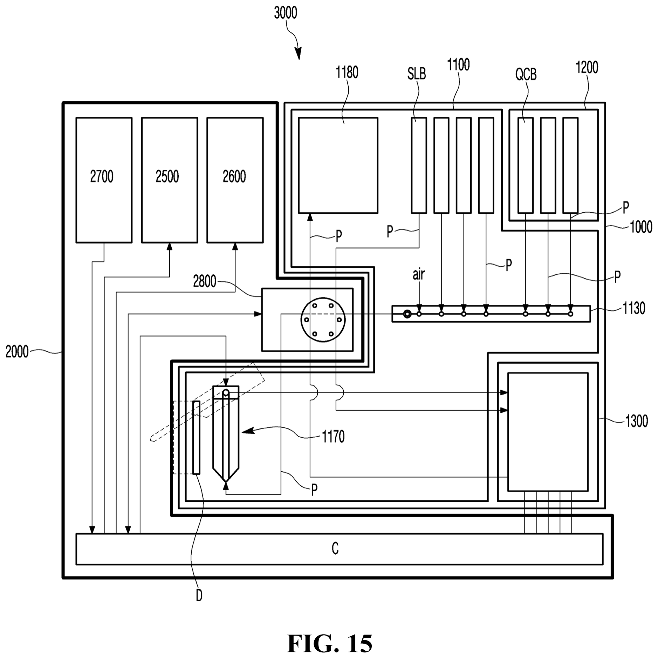

FIG. 1 is a diagram schematically illustrating a front surface side of a cartridge assembly for blood analysis according to an embodiment of the present disclosure. FIG. 2 is a diagram schematically illustrating a rear surface side of a cartridge assembly for blood analysis according to an embodiment of the present disclosure. FIGS. 3 and 4 are diagrams each schematically illustrating a separated view of a main cartridge and a sub-cartridge of a cartridge assembly for blood analysis according to an embodiment of the present disclosure. FIG. 5 is a diagram schematically illustrating a coupled cross-section of an insert connector of a main cartridge and an accommodation connector of a sub-cartridge in a cartridge assembly for blood analysis according to an embodiment of the present disclosure. FIG. 6 is a diagram schematically illustrating a separated view of a main cartridge and a sensor card of a cartridge assembly for blood analysis according to an embodiment of the present disclosure. FIG. 7 is a diagram schematically illustrating a coupled view of an accommodation connector of a main cartridge and a sensor card in a cartridge assembly for blood analysis according to an embodiment of the present disclosure. FIG. 8 is a diagram illustrating a part of a cross-section along line I-I' of FIG. 2. FIG. 9 is a diagram schematically illustrating a blood analysis device according to an embodiment of the present disclosure. FIG. 10 is a diagram schematically illustrating a blood analysis device main body applied to a blood analysis device according to an embodiment of the present disclosure. FIG. 11 is a diagram schematically illustrating an exploded view of some components of a blood analysis device main body applied to a blood analysis device according to an embodiment of the present disclosure. FIG. 12 is a diagram schematically illustrating a cartridge assembly pipeline opening/closing structure of a blood analysis device according to an embodiment of the present disclosure. FIG. 13 is a diagram schematically illustrating an operating structure of a specimen supply part and an opening/closing door of a blood analysis device according to an embodiment of the present disclosure. FIG. 14 is a diagram schematically illustrating a cartridge assembly discharge structure of a blood analysis device according to an embodiment of the present disclosure. FIG. 15 is a diagram schematically illustrating a connection relationship between components of a blood analysis device according to an embodiment of the present disclosure. In this regard, in each drawing, some components are omitted to clearly illustrate the structure of the cartridge assembly blood analysis device main body and the blood analysis device.

Meanwhile, for convenience of explanation, hereinafter, the "cartridge assembly for blood analysis" will be referred to as the "cartridge assembly" and the "blood analysis device main body" will be referred to as the "main body."

Referring to FIGS. 1 to 15, a blood analysis device 3000 according to an embodiment of the present disclosure includes a cartridge assembly 1000 and a main body 2000.

The cartridge assembly 1000 may be a replacement part that is inserted into an accommodation space 2200 of the main body 2000 and used for a certain period of time before being replaced. The cartridge assembly 1000 includes a main cartridge 1100, a sub-cartridge 1200, and a sensor card 1300.

The main cartridge 1100 forms the overall outer shape of the cartridge assembly 1000. In this sense, in the description below, one surface of the cartridge assembly 1000 and one surface of the main cartridge 1100 may be used interchangeably, and the other surface of the cartridge assembly 1000 and the other surface of the main cartridge 1100 may be used interchangeably. In addition, in the description below, one surface of the cartridge assembly 1000 and one surface of the main cartridge 1100 refer to the front surface of the cartridge assembly 1000 and the front surface of the main cartridge 1100 based on the directions of FIGS. 3 and 6. In addition, in the description below, the other surface of the cartridge assembly 1000 and the other surface of the main cartridge 1100 refer to the front surface of the cartridge assembly 1000 and the front surface of the main cartridge 1100 based on the direction of FIG. 1. In addition, one surface of the main cartridge 1100 may refer to a part that forms an overall planar shape among the outlines of the main cartridge 1100 illustrated on the front surface based on FIGS. 3, 4, and 6.

The main cartridge 1100 includes a main case 1110, a reagent solution bag SLB, a supply and cut-off part 1120, a solution distribution part 1130, a pipeline P, a handle H1, a leakage receiving part 1140, an insert connector 1150, an accommodation connector 1160, a specimen supply part 1170, an opening/closing door D, and a specimen disposal bag 1180.

The main case 1110 forms the outer shape of the main cartridge 1100. In this sense, in the description below, one surface of the main cartridge 1100 and one surface of the main case 1110 may be used interchangeably, and the other surface of the main cartridge 1100 and the other surface of the main case 1110 may be used interchangeably. For example, the main case 1110 may be a form in which an upper case and a lower case are coupled with each other, but the scope of the present disclosure is not limited thereto. The main case 1110 may be formed in a rectangular box shape overall. The main case 1110 may form an internal space so that the reagent solution bag SLB, the specimen supply part 1170, the pipeline P, etc., which will be described later, may be disposed therein.

The reagent solution bag SLB is disposed inside the main case 1110. A plurality of reagent solution bags SLB may be disposed inside the main case 1110. Different types of reagent solutions may be contained in the plurality of reagent solution bags SLB. The plurality of reagent solutions may be transferred to the sensor card 1300 to be described later and used as a standard for correcting the measured concentration of electrolytes, gases, and metabolites, or the hematocrit, etc., contained in a specimen (e.g., blood) supplied through the specimen supply part 1170 to be described later. The reagent solutions of the plurality of reagent solution bags SLB may be connected to the solution distribution part 1130 through the plurality of pipelines P. As the reagent solution bags, conventional bags made of synthetic resin or aluminum may be used. Meanwhile, the reagent solution of any one of the plurality of reagent solution bags SLB may be directly supplied to the sensor card 1300 through the pipeline P without passing through the solution distribution part 1130.

A valve (see FIG. 8) is disposed on one side of the reagent solution bag SLB. The valve (see FIG. 8) of the reagent solution bag SLB is connected to the main case 1110 through a valve coupling hole 1113 formed on one surface of the main case 1110 and is exposed to the outside of the main case 1110. As a result, only the valves that require manipulation from the outside of the main cartridge 1100 may be exposed. As a result, the reagent solution in the reagent solution bag SLB may be preserved and utilized more efficiently. Specifically, the cartridge assembly 1000 may be provided to the user with the valve of the reagent solution bag SLB closed, and the user may allow the inflow of the reagent solution in the reagent solution bag SLB through the pipeline P by opening the reagent solution bag SLB valve from the outside of the cartridge assembly 1000 only when the cartridge assembly 1000 is inserted into the accommodation space 2200 of the main body 2000.

The supply and cut-off parts 1120 close or open the plurality of pipelines P connected to a plurality of inlets of the solution distribution part 1130. In addition, the supply and cut-off part 1120 closes or opens a pipeline P that is not connected to the solution distribution part 1130 but is directly connected to the sensor card 1300. The reagent solution in the reagent solution bag SLB, the quality control solution in the quality control solution bag QCB to be described later, and air are supplied through the plurality of pipelines P to the plurality of inlets of the solution distribution part 1130, and the supply and cut-off part 1120 may selectively supply the reagent solution, the quality control solution, and the air to the sensor card 1300 to be described later by closing or opening each of the plurality of pipelines P. The supply and cut-off part 1120 includes an elastic member 1121 and a pressure member 1122. The pressure member 1122 includes a body part 1122a having a substantially cylindrical shape and an extension part 1122b having a flat shape and extending from the body part 1122a. A pipeline insertion hole 1122c into which the pipeline P is inserted is formed in the extension part 1122b. The pressure member 1122 penetrates one surface of the main case 1110 and is disposed in a form that is slidable with respect to one surface of the main case 1110. The elastic member 1121 is disposed between the first direction 1 end part of the pressure member 1122 and the main case 1110 to elastically support the pressure member 1122. The elastic member 1121 may provide an elastic force that presses the pressure member 1122 in the first direction 1. The elastic member 1121 may be a spring, but the scope of the present disclosure is not limited thereto. In the above-described structure, the pipeline P is pressed by the elastic force of the elastic member 1121 along the first direction 1 in a state being inserted into the pipeline insertion hole 1122c formed in the extension part 1122b of the pressure member 1122. Due to this, the movement of the reagent solution, quality control solution, and air within the pipeline P is restricted. Meanwhile, when the cartridge assembly 1000 is inserted into the accommodation space 2200 of the main body 2000, the supply and cut-off part 1120 is disposed to face the pressure part 2400 of the main body 2000 (see FIG. 12). In this situation, when the pressure part 2400 presses the supply and cut-off part 1120 in the opposite direction to the first direction 1 with a force greater than the elastic force of the elastic member 1121, the elastic member 1121 is compressed, and the pressure member 1122 slides in the opposite direction to the first direction 1 on one surface of the main case 1110. As a result, the pressurized state of the pipeline P is released, and the movement of the reagent solution, quality control solution, and air within the pipeline P is achieved.

The solution distribution part 1130 is disposed on one surface of the main case 1110. The solution distribution part 1130 includes an integrated pipeline 1131, an inlet 1132, and an outlet 1133. A plurality of inlets 1132 are formed in the integrated pipeline 1131, and a single outlet 1133 is formed in the integrated pipeline 1131. The plurality of inlets 1132 are connected to the reagent solution, the quality control solution, and air through the pipeline P. The outlet 1133 is connected to a sensor card 1300 to be described later through the pipeline P. According to the structure of the solution distribution part 1130 and the operation of the supply and cut-off part 1120 described above and a driving part 2400, the reagent solution, the quality control solution, and the air may be selectively introduced into the integrated pipeline 1131 through the inlet 1132 and provided to the sensor card 1300 through the outlet 1133.

The integrated pipeline 1131 is formed in a form extending in a second direction 2. The plurality of inlets 1132 and the single outlet 1133 are formed in the integrated pipeline 1131 in a form spaced apart from each other along the second direction 2. In the conventional case, the solution distribution part has been formed in a form in which the inlets are generally spaced apart from each other along the circumference, so there was a limit to increasing the number of inlets. Due to this, in the conventional case, the number of solution bags that may be disposed in the cartridge was limited. In the present embodiment, by disposing the integrated pipeline 1131 in a substantially straight shape, the number of inlets 1132 may be relatively easily increased. As a result, the cartridge assembly 1000 according to the present embodiment may relatively easily increase the number of reagent solution bags SLB and quality control solution bags QCB included inside.

The handle H1 may be coupled to the main case 1110 in a rotatable form at one end part. In addition, the handle H1 may be elastically rotated through an elastic member such as a spring. The handle H1 may be coupled to the upper surface of the main case 1110. In this case, a handle accommodation groove 1117 may be formed on the upper surface of the main case 1110. Referring to FIG. 1, in a normal state, the handle H1 is elastically supported by the elastic member and disposed to form an acute angle with the upper surface of the main case 1110 (see the handle H1 illustrated in a solid line in FIG. 1). In a state where a user holds the handle H1 for transporting the main cartridge 1100 or cartridge assembly 1000, the handle H1 may be rotated and disposed to form an angle close to a right angle with the upper surface of the main case 1110 (see the handle H1 illustrated in a dotted line in FIG. 1). Meanwhile, the handle H1 is pressed in the opposite direction to a third direction 3 by the frame F of the main body 2000 when the cartridge assembly 1000 is inserted into the accommodation space 2200 of the main body 2000 and is completely accommodated in the handle accommodation groove 1117 (see FIG. 9). In addition, when the cartridge assembly 1000 is discharged from the accommodation space 2200 of the main body 2000, the handle H1 is released from the pressure by the frame F and elastically rotated from the upper surface of the main case 1110 to protrude from the handle accommodation groove 1117. The handle H1 may improve the handleability of the main cartridge 1100 and/or the cartridge assembly 1000.

The leakage receiving part 1140 is formed on the lower side of the valve coupling hole 1113 in one surface of the main case 1110. When the reagent solution leaks out of the main case 1110 through the valve of the reagent solution bag SLB, the leakage receiving part 1140 prevents the leakage from falling from the outside of the main case 1110 toward the lower surface side of the main case 1110. In other words, leakage may occur from the valve due to tolerance or defects when the valve of the reagent solution bag SLB is inserted and coupled to the valve coupling hole 1113, and the leakage receiving part 1140 is formed on the lower side of the valve coupling hole 1113 to prevent the leakage from the valve from falling to the lower surface of the main case 1110. When the cartridge assembly 1000 is inserted into the accommodation space 2200 of the main body 2000, the main case 1110 is also inserted into the main body 2000, and the leakage receiving part 1140 prevents leakage from penetrating into the main body 2000.

Referring to FIG. 8, the leakage receiving part 1140 includes a support plate 1141 and a partition wall 1142. The support plate 1141 may be formed to protrude in the first direction 1 from one surface of the main case 1110 and may be in the form of a plate extending along the second direction 2. The partition wall 1142 is formed to extend in the third direction 3 from the first direction 1 end part, the second direction 2 end part, and the end part of the opposite direction to the second direction 2 of the support plate 1141. The partition wall 1142 prevents the leakage from splashing out of the support plate 1141 when the bottom surface of the support plate 1141 comes into contact with the leakage.

The bottom surface level of the support plate 1141 may become lower from the first direction 1 end part of the support plate 1141 toward one surface of the main case 1110. Due to the bottom surface level of the support plate 1141, the leakage that has fallen to the bottom surface of the support plate 1141 does not stagnate in the support plate 1141, but may move to the inner bottom surface of the main case 1110 through the leakage movement hole 1115 formed on one surface of the main case 1110. Meanwhile, the level of the inner bottom surface of the main case 1110 also becomes lower from one surface of the main case 1110 toward the other surface of the main case (the opposite direction to the first direction 1), so that the leakage moved into the inside of the main case 1110 moves toward the other surface side of the main case 1110 along the inner bottom surface of the main case 1110. Since the other surface of the main case 1110 is the surface exposed to the user side when the main case 1110 is inserted into the accommodation space 2200 of the main body 2000, the user may detect the leakage of the reagent solution relatively quickly.

The leakage receiving part 1140 may be formed in a separate form at the lower part of each of the plurality of valve coupling holes 1113. Alternatively, the leakage receiving part 1140 may include first and second leakage receiving parts spaced apart in the second direction 2. Here, the first leakage receiving part may be formed at the lower part of any one of the plurality of valve coupling holes 1113, and the second leakage receiving part may extend in the second direction 2 to cover the lower parts of all of the remaining valve coupling holes 1113 and may be spaced apart from the first leakage receiving part in the second direction 2. Alternatively, the leakage receiving part 1140 may be formed in a single shape that covers the lower parts of all of the plurality of valve coupling holes 1113.

In addition, a leakage passage hole through which leakage passes may be formed in a region on the other surface side of the inner bottom surface of the main case 1110, and a discharge hole communicating with the leakage passage hole may be formed in a region of the main body 2000 corresponding to the leakage passage hole of the main case 1110. In this case, the leakage from the valve may be discharged to the outside of the main body 2000 through the path of the leakage receiving part 1140, the leakage movement hole 1115, the bottom surface of the main case 1110, the leakage passage hole, and the discharge hole of the main body 2000.

The insert connector 1150 connects the quality control solution of the quality control solution bag QCB of the sub-cartridge 1200 to the solution distribution part 1130 of the main cartridge 1100. The accommodation connector 1160 connects the outlet 1133 of the solution distribution part 1130 and the sensor card 1300. The insert connector 1150 and the accommodation connector 1160 will be described later.

The specimen supply part 1170 transfers a specimen (e.g., blood) to the sensor card 1300, or transfers a reagent solution, quality control solution, or air that has passed through the solution distribution part 1130 to the sensor card 1300. The specimen supply part 1170 may be rotatably coupled to the frame disposed within the main case 1110 inside the main case 1110 on the other surface side of the main case 1110. When no specimen is supplied, the specimen supply part 1170 may be disposed inside the main case 1110 and may not be exposed to the outside since the opening/closing door D to be described later is closed. When a specimen is supplied, the specimen supply part 1170 may be rotated by the driving part 2300 of the main body 2000 to push the opening/closing door D and be exposed to the outside of the other surface side of the main case 1110.

The specimen supply part 1170, as illustrated in FIG. 13, includes a first rotating body 1171 that is coupled to the driving part 2300 of the main body 2000 to rotate, a guide 1172 having one end part coupled to the first rotating body 1171, a second rotating body 1173 that is rotatably coupled to the frame of the main case 1110 and slides against a slit of the guide 1172 when rotated, and a needle 1174 that is coupled to the second rotating body 1173 to slide against the slit of the guide 1172. The second rotating body 1173 includes a main body part 1173a that is rotatably coupled to the frame of the main case 1110 and a slide part 1174b that is disposed within the slit of the guide 1172 and is respectively connected to the needle 1174 and the pipeline P. When the specimen is not supplied, the needle 1174 does not protrude from the guide 1172 and fluidly connects the outlet 1133 of the solution distribution part 1130 and the sensor card 1300. Hereinafter, the operating state of the specimen supply part 1170 when the specimen is supplied will be described. When the first rotating body 1171 is rotated by the driving part 2300 of the main body 2000, the guide 1172 connected to the first rotating body 1171 is rotated. Meanwhile, since the main body part 1173a of the second rotating body 1173 is rotatably coupled to the frame, the main body part 1173a of the second rotating body 1173 is also rotated. At this time, the slide part 1173b of the second rotating body 1173 disposed within the slit of the guide 1172 slides relatively to the slit of the guide 1172. Accordingly, the needle 1174 coupled with the slide part 1173b slides relatively to the slit of the guide 1172. Meanwhile, the main body part 1173b of the second rotating body 1173 is formed to extend in the opposite direction of the third direction 3 from the rotation axis, and the main body part 1173b is rotated to open the opening/closing door D disposed on the other surface of the main case 1110. By this mechanism, the opening/closing door D is passively opened, and the needle 1174 is exposed to the outside of the other surface of the main case 1110.

The opening/closing door D is elastically and rotatably coupled to the other surface of the main case 1110. For example, an elastic member that provides elastic force in the direction in which the opening/closing door D closes may be coupled to the opening/closing door D. Accordingly, even if an external force is applied to open the opening/closing door D, when the external force is removed, the opening/closing door D closes automatically by the elastic force of the elastic member. This opening/closing door D protects the components disposed inside the main case 1110 from foreign substances. In particular, the opening/closing door D prevents foreign substances from penetrating the needle 1174, and since the passive opening and closing of the opening/closing door D by the operation of the driving part 2300 has been described above, a detailed description thereof will be omitted.

The specimen disposal bag 1180 may be disposed within the main case 1110 together with the reagent solution bag SLB. The specimen disposal bag 1180 may be connected to the sensor card 1300 to collect reagent solutions or the like, used in the sensor card 1300.

The sub-cartridge 1200 is a physically distinct configuration from the main cartridge 1100, and is inserted into the sub-cartridge accommodation groove 1111 of the main case 1110 in a form that is detachable from the main cartridge 1100.

The sub-cartridge 1200 includes a sub-case 1210, a quality control solution bag QCB, and an accommodation connector 1220.

The sub-case 1210 forms the exterior of the sub-cartridge 1200. In this sense, in the description below, there may be cases where one surface of the sub-cartridge 1200 and one surface of the sub-case 1210 are used interchangeably, and the other surface of the sub-cartridge 1200 and the other surface of the sub-case 1210 are used interchangeably. The sub-case 1210 may be, for example, a form in which an upper case and a lower case are coupled to each other, but the scope of the present disclosure is not limited thereto. The sub-case 1210 may be formed to have a rectangular cross-section in which the length along the second direction 2 is smaller than the length along the third direction 3. The sub-case 1210 forms an internal space, so that a quality control solution bag QCB and a pipeline P, which will be described later, are disposed therein. The accommodation connector 1220 for fluid connection with the main cartridge 1100 is formed on one side of the sub-case 1210.

The sub-case 1210 has one surface (the front surface of the sub-case 1210 based on the direction of FIG. 3) and the other surface (the rear surface of the sub-case 1210 based on the direction of FIG. 3). The sub-cartridge 1200 is inserted into the sub-cartridge accommodation groove 1111 after being disposed so that the other surface of the sub-case 1210 faces one surface of the main cartridge 1100.

The sub-case 1210 inserted into the sub-cartridge accommodation groove 1111 may not be exposed to the outside at the remaining surfaces except for one surface exposed to one surface of the main cartridge 1110. In addition, the sub-case 1210 inserted into the sub-cartridge accommodation groove 1111 may not protrude further outward than the main case 1110. For example, the three-dimensional shape of the sub-case 1210 may be aligned with the three-dimensional shape of the sub-cartridge accommodation groove 1111 formed in the main case 1110. In this case, one surface of the main cartridge 1100 and one surface of the sub-cartridge 1200 may be located on substantially the same plane. Due to the shapes of the sub-cartridge 1200 and the sub-case 1210 described above, the blood analysis device 3000 according to the present embodiment may not experience a change in outer shape even when the sub-cartridge 1200 is used, compared to a conventional technology that involves a change in outer shape when using a sub-cartridge. In addition, the cartridge assembly 1000 according to the present embodiment may accommodate the sub-cartridge 1200 inside without increasing the cross-sectional area of the main cartridge 1100. Therefore, the overall outer shape of the cartridge assembly 1000 including the sub-cartridge 1200 may not change significantly from the outer shape of the main cartridge 1100.

A handle H2 protruding from one surface of the sub-case 1210 may be formed on one surface of the sub-case 1210. The handle H2 of the sub-case 1210 may improve the handleability and transportability of the sub-cartridge 1200. This handle H2 may also be formed so as not to protrude further than the end part of the main cartridge 1100 in the first direction 1.

The quality control solution bag QCB is disposed inside the sub-case 1210. A plurality of quality control solution bags QCB may be disposed inside the sub-case 1210. Different types of quality control solutions may be contained within the plurality of quality control solution bags QCB. The quality control solution is connected to the sensor card 1300 to be described later, and the quality control solution may be used for automatic testing that periodically checks the measurement of the sensor card 1300. The values measured through the quality control solution are stored as data, so that the user may check the status of the equipment. The quality control solutions of the plurality of quality control solution bags QCB are connected to the accommodation connector 1220 to be described later through a plurality of pipelines P. When the sub-cartridge 1200 is inserted into the main cartridge 1100, the accommodation connector 1220 of the sub-cartridge 1200 and the insert connector 1150 of the main cartridge 1100 may be fluidly connected, so that the quality control solution may be connected to the solution distribution part 1130. The quality control solution bag QCB may be a conventional bag made of synthetic resin or aluminum.

A valve (not shown) is disposed on one side of the quality control solution bag QCB, similar to the valve of the reagent solution bag SLB (see FIG. 8). The valve of the quality control solution bag QCB is coupled to the sub-case 1210 through a valve coupling hole formed on one surface of the sub-case 1210 and is exposed to the outside of the sub-case 1210. Due to this, only the valves requiring manipulation from the outside of the quality control solution bag QCB may be exposed. As a result, the quality control solution in the quality control solution bag QCB may be more efficiently preserved and utilized. Specifically, the cartridge assembly 1000 may be provided to the user with the valve of the quality control solution bag QCB closed, and the user may allow the inflow of the quality control solution within the quality control solution bag QCB into the pipeline P by opening the valve of the quality control solution bag QCB from the outside of the cartridge assembly 1000 only when the cartridge assembly 1000 is inserted into the accommodation space 2200 of the main body 2000.

Meanwhile, a configuration corresponding to the leakage receiving part 1140 described in the main cartridge 1100 may also be formed in the sub-cartridge 1200.

The accommodation connector 1220 of the sub-cartridge 1200 and the insert connector 1150 of the main cartridge 1100 are configurations for fluid connection between the main cartridge 1100 and the sub-cartridge 1200. Referring to FIGS. 3 to 5, the accommodation connector 1220 of the sub-cartridge 1200 is disposed in one region of the sub-case 1210. The insert connector 1150 of the main cartridge 1100 is disposed in one region of the sub-cartridge accommodation groove 1111 corresponding to the accommodation connector 1220. A plurality of accommodation holes 1220H are formed in the accommodation connector 1220. A plurality of protrusion parts 1150P to be inserted into the plurality of accommodation holes 1220H are formed in the insert connector 1150. When the sub-cartridge 1200 is inserted into the main cartridge 1100, the accommodation connector 1220 moves forward toward the insert connector 1150, and as a result, the protrusion parts 1150P of the insert connector 1150 are inserted into the accommodation holes 1220H of the accommodation connector 1220. The plurality of accommodation holes 1220H are connected to the plurality of quality control solution bags QCB through the pipeline P of the sub-cartridge 1200, and the protrusion part 1150P is connected to the inlet 1132 of the solution distribution part 1130 through the pipeline P of the main cartridge 1100, and by inserting the protrusion part 1150P into the accommodation hole 1220H, the quality control solution inside the sub-cartridge 1200 is connected to the solution distribution part 1130 of the main cartridge 1100, which is a component outside the sub-case 1210. Meanwhile, by forming an accommodation hole 1220H in the sub-cartridge 1200 and forming the protrusion part 1150P in the main cartridge 1100, damage to the connection configuration for fluid connection between the main cartridge 1100 and the sub-cartridge 1200 may be prevented.

A curved pipe may be fixedly disposed inside the insert connector 1150 and the accommodation connector 1220. This curved pipe may prevent a defect caused by a bend in the pipeline of the connection part when the main cartridge 1100 and the sub-cartridge 1200 are fluidly connected.

Meanwhile, the above-described sub-cartridge 1200 may be omitted from the cartridge assembly 1000 depending on the case. In other words, the cartridge assembly 1000 according to the present embodiment may be a form in which the main cartridge 1100, the sub-cartridge 1200, and the sensor card 1300 to be described later are coupled, or may be a form composed only of the main cartridge 1100 and the sensor card 1300. Specifically, in an environment where the quality control of the blood analysis device 3000 according to the present embodiment is automatically performed by the main body 2000, the sub-cartridge 1200 may be inserted into the main body 2000 in a state of being inserted into the main cartridge 1100. As another example, in an environment where the quality control of the blood analysis device 3000 according to the present embodiment is manually performed by a user or the like, the sub-cartridge 1200 may not be inserted into the main cartridge 1100.

The sensor card 1300 may measure the concentration of electrolytes, gases, and metabolites contained in blood or the hematocrit by passing a specimen through it and transmit the measured values to a controller C.

The sensor card 1300 is detachably coupled to the main cartridge 1100. The sensor card 1300 is coupled to the main cartridge 1100 by being at least partially inserted into the sensor card accommodation connector 1160 of the main cartridge 1100 disposed in the main case 1110. The sensor card accommodation connector 1160 has a plurality of accommodation holes 1160H. Each of the plurality of accommodation holes 1160H is connected to the outlet 1133 of the solution distribution part 1130, any one of the plurality of reagent solutions that is not connected to the inlet 1132 of the solution distribution part 1130, and the specimen disposal bag 1180. In this structure, by inserting the protrusion part 1300P of the sensor card 1300 into the plurality of accommodation holes 1160H of the sensor card accommodation connector 1160, the outlet 1133 of the solution distribution part 1130, any one of the plurality of reagent solutions that is not connected to the inlet 1132 of the solution distribution part 1130, and the specimen disposal bag 1180 are each fluidly connected to the sensor card 1300.

The main body 2000 constitutes the blood analysis device 3000 according to the present embodiment together with the cartridge assembly 1000 described above.

Referring to FIGS. 9 to 11 and FIG. 15, the main body 2000 includes a frame F, a case 2100, a driving part 2300, a pressure part 2400, a discharge part 2500, a controller C, an output part 2600, an input part 2700, and a transport part 2800.

The frame F forms a space inside the case 2100 so that various components of the main body 2000 to be described later are coupled. In the following description, components installed in the frame F have the same meaning as those installed inside the case 2100 and are not limited to the examples illustrated in the drawings. Meanwhile, a handle H3 is coupled with the case 2100 of the main body 2000 so that the user may easily carry the blood analysis device 3000 according to the present embodiment to the installation location.

The frame F forms the frame of the main body 2000 and is provided with the accommodation space 2200 into which the cartridge assembly 1000 may be inserted and fixed at a certain location. The controller C and the output part 2600 controlled by the controller C are coupled with the frame F. The output part 2600 is a display device that displays data processed by the controller C so that a user may recognize the data, and includes a typical display 2610 and a printing device 2620. The display 2610 may be, for example, a touch screen, in which case the display 2610 may be formed integrally with the input part 2700. The output part 2600 is formed integrally with the main body 2000. The output part 2600 is not limited to the above-described example, and may include a typical data output port (not shown) that is electrically connected to a computer or the like to transmit and receive data.

The driving part 2300 exposes the aforementioned specimen supply part 1170 to the outside of the cartridge assembly 1000 by rotational force, or disposed the specimen supply part 1170 inside the cartridge assembly 1000. The driving part 2300 includes a connecting member 2310 and a driving member 2320 that rotates the connecting member 2310 about an axis. It is preferable that the connecting member 2310 be provided with a protrusion part at the front end part so as to transmit rotational force to the specimen supply part 1170. When the cartridge assembly 1000 is inserted into the accommodation space 2200 of the frame F, the connecting member 2310 moves along a guide groove formed on the side surface of the cartridge assembly 1000 and is fitted into the first rotating body 1171 of the specimen supply part 1170. The driving part 2300 is not limited to the structure described in the embodiment of the present disclosure, and may be designed to be formed only with gears without a belt or with an appropriate power transmission structure such as linear motion, as needed, and may be manufactured in various configurations.

The pressure part 2400 supplies or blocks the reagents provided to the cartridge assembly 1000 to the sensor card 1300. The pressure part 2400 is driven by the control of the controller C. The pressure part 2400 includes a moving member 2410 and a driving member 2420 that drives the moving member 2410 in the first direction 1 and the opposite direction to the first direction. The pressure part 2400 may be formed in a number corresponding to the supply and cut-off parts 1120. The pressure part 2400 is controlled by the controller C. The pressure part 2400 may be applied as an embodiment of the present disclosure as a structure capable of linear movement through rotational movement, as long as the moving member 2410 may be moved to pressurize and move the supply and cut-off part 1120. A pressure part operation detection sensor that may detect the operating status of the pressure part 2400 and transmit the signal to the controller C may be installed on one side of the pressure part 2400.

The discharge part 2500 discharges the cartridge assembly 1000 inserted into the accommodation space 2200 of the frame F to the outside of the accommodation space 2200. The discharge part 2500 is driven by the control of the controller C. The discharge part 2500 includes a rotating member 2510 and a driving member 2520 that rotates the rotating member 2510. The rotating member 2510 has a shape extending in the third direction 3 from the rotation axis. Due to the shape of the rotating member 2510, the rotating member 2510 pushes the cartridge assembly 1000 in the opposite direction to the first direction 1 when rotating.

The transport part 2800 transports fluid under the control of the controller C. Here, the fluid transported by the transport part 2800 includes a fluid flowing through the pipeline P connecting the outlet 1133 of the solution distribution part 1130 and the sensor card 1300, a fluid flowing through the pipeline P connecting the reagent solution bag SLB that is not connected to the solution distribution part 1130 among the plurality of reagent solution bags SLB and the sensor card 1300, and a fluid flowing through the pipeline P connecting the sensor card 1300 and the specimen disposal bag 1180. The transport part 2800 may be configured as a structure in which a rotating body driven by a motor is in close contact with the pipeline P and rotates to transport fluid. The transport part 2800 is not limited to the above-described example, and a conventional one may be used, and a pump or the like capable of transporting fluid may also be used.

The controller C controls the driving part 2300, the pressure part 2400, the discharge part 2500, the output part 2600, and the transport part 2800. The controller C may control the above configuration by a preset algorithm or by a user's manipulation of the input part 2700. In addition, the controller C may receive a signal measured by the sensor card 1300 and process the same. Meanwhile, it is preferable that the controller that receives and processes the signal detected by the sensor card 1300 and the controller that controls the driving part 2300, the pressure part 2400, etc., be electrically separated. In other words, in order to avoid electrical interference between the signal processing part detected in the sensor card 1300 and the part driving the pump-solenoid, it is preferable to apply an isolation circuit that configures the input power separately and also configures the input and output signal parts separately. In addition, it is preferable that the signals detected in the controller that receives and processes the signals detected in the sensor card 1300 be electrically isolated according to their types. In other words, it is preferable to apply an electrical isolation circuit to avoid individual sensing signals for each measurement item of the signals, i.e., electrolytes, gas species, metabolites, and hematocrit detected in the sensor card 1300, or electrical interference.

The main body 2000 may further include a heater block equipped with a heater to maintain the temperature of the sensor card 1300 constant. It is preferable that such a heater block is electrically connected so as to be operated under the control of the controller C. In addition, the main body 2000 may further include a cartridge assembly locking device controlled by the controller C to maintain the cartridge assembly 1000 in a coupled state, and a cartridge door lock detection sensor that detects whether the cartridge assembly locking device maintains a locked state and transmits the signal to the controller C.

Hereinafter, an exemplary operation process of the blood analysis device 3000 according to an embodiment of the present disclosure will be described.

First, the user inserts the cartridge assembly 1000 into the accommodation space 2200 of the main body 2000. Then, the controller C checks whether the sensor card 1300 is normally inserted into the cartridge assembly 1000. Then, the controller C locks the cartridge assembly 1000 by the cartridge assembly locking device. At this time, the cartridge door lock detection sensor senses a signal as to whether the cartridge assembly locking device is locked, and the controller C receives the signal sensed by the cartridge door lock detection sensor.

Thereafter, a pre-warm-up for analysis is performed. **In** the warm-up process, the reagent solution of the reagent solution bag SLB, the quality control solution of the quality control solution bag QCB, and air are transported alternately to first measure the reference data. This reference data is subsequently used to appropriately correct the data measured from the specimen.

The process of transporting the reagent solution, the quality control solution, and the air is described as follows. The controller C controls the pressure part 2400 to control the moving member 2410 that may open the pipeline P (meaning the pipeline connected to each of the plurality of reagent solution bags SLB, the pipeline connected to each of the plurality of quality control solution bags QCB, and the pipeline connected to the air) to be transported. Then, the moving member 2410 pressurizes the pressure member 1122 of the supply and cut-off part 1120 while moving in the opposite direction to the first direction 1. Then, the pressure member 1122 moves in the opposite direction to the first direction 1 while overcoming the elastic force of the elastic member 1121. Then, the state in which the pressure member 1122 was pressing the pipeline P is released. Accordingly, the reagent solution, the quality control solution, and the air are transported along the pipeline P. The reagent solutions, excluding any one of those accommodated in the plurality of reagent solution bags SLB, the quality control solution, and the air are transferred to the sensor card 1300 through the outlet 1133 of the solution distribution part 1130 and the specimen supply part 1170. Any one of the plurality of reagent solution bags SLB is not connected to the solution distribution part 1130 and is directly transferred to the sensor card 1300. An electrical signal according to the fluid transferred to the sensor card 1300 is transmitted to the controller C. The fluid supplied to the sensor card 1300 flows into the specimen disposal bag 1180 and is stored there.

When the specimen is ready for measurement, the controller C controls the driving part 2300 to expose the specimen supply part 1170 to the outside. Specifically, when the connecting member 2310 is rotated using the driving member 2320, the first rotating body 1171 connected to the connecting member 2310 also rotates. The guide 1172 coupled to the first rotating body 1171 and the second rotating body 1172 coupled to the guide 1172 rotate due to the rotation of the first rotating body 1161. At this time, the slide part 1174b of the second rotating body 1172 slides within the slit of the guide 1172 so that the needle 1174 is exposed to the outside. Then, the user contacts the specimen sample with the tip of the needle. Then, the controller C controls the transport part 2800 to transport the specimen sample to the inside of the sensor card 1300. When the user manipulates the input part 2700, the controller C controls the driving part 2300 to return the specimen supply part 1170 to its original location. Continuously, the sensor card 1300 measures electrical signals according to the concentration of electrolytes, gases, and metabolites in the blood, or hematocrit, and transmits the signals to the controller C. In addition, the controller C calculates the analysis results of the measured substances based on the data measured by the reagent solutions accommodated in the reagent solution bag SLB, and stores the data in a storage device (not shown) or outputs the data to the output part 2600. In addition, the sensor card 1300 is cleaned with the reagent solution in the reagent solution bag SLB that is not connected to the solution distribution part 1130 among the reagent solution bags SLB. Cleaning the sensor card 1300 means that the controller C drives the transport part 2800 so that the reagent solution in the reagent solution bag SLB that is not connected to the solution distribution part 1130 among the reagent solution bags SLB passes through the sensor card 1300 and flows into the specimen disposal bag 1180. In addition, the controller (C) may perform an analysis assay.

The protection scope of the present disclosure is not limited to the description and expression of the embodiments explicitly described above. In addition, it is added once again that the protection scope of the present disclosure may not be limited due to obvious changes or substitutions in the technical field to which the present disclosure belongs.

### <Explanation of Symbols>

1000: Cartridge assembly
1100: Main cartridge
1110: Main case
1120: Supply and cut-off part
1130: Solution distribution part
1140: Leakage receiving part
1150: Insert connector
1160: Accommodation connector
1170: Specimen supply part
1180: Specimen disposal bag
1200: Sub-cartridge
1210: Sub-case
1220: Accommodation connector
1300: Sensor card
2000: Blood analysis device main body
2100: Case
2300: Driving part
2400: Pressure part
2500: Discharge part
2600: Output part
2700: Input part
2800: Transport part
C: Controller
D: Opening/closing door
F: Frame
H1, H2, H3: Handle
P: Pipeline

## Claims

1. A blood analysis device comprising:
a blood analysis device main body comprising a frame in which an accommodation space is formed; and
a cartridge assembly which is inserted into the accommodation space and comprises a main cartridge comprising a main case, a plurality of reagent solution bags disposed inside the main case, and a specimen supply part disposed inside the main case, a sub-cartridge which is detachably inserted into a sub-cartridge accommodation groove formed on one surface of the main cartridge and comprises a plurality of quality control solution bags disposed therein, and a sensor card detachably coupled to the main cartridge,
wherein the cartridge assembly is inserted into the blood analysis device main body so that one surface of the main case faces the accommodation space of the frame, and
the main cartridge further comprises an opening/closing door which is elastically and rotatably coupled to another surface of the main case facing the one surface of the main case and is opened and closed by the specimen supply part.

2. The blood analysis device of claim 1, wherein the blood analysis device main body further comprises a driving part coupled to the frame,
the specimen supply part comprises a first rotating body coupled to the driving part to rotate, a guide having one end part coupled to the first rotating body, a second rotating body coupled to the guide and configured to slide in a slit formed in the guide, and a needle coupled to the second rotating body, and
the second rotating body presses to open the opening/closing door when rotating.

3. The blood analysis device of claim 1, wherein the blood analysis device main body further comprises a discharge part coupled to the frame and configured to rotate to discharge the cartridge assembly from the accommodation space of the frame.

4. The blood analysis device of claim 1, wherein a handle accommodation groove is formed on an upper surface of the main cartridge connected to the one surface of the main cartridge,
the cartridge assembly further comprises a handle having one end part which is elastically and rotatably coupled to the upper surface of the main cartridge, and
the handle is accommodated in the handle accommodation groove by being pressed by the frame when the cartridge assembly is inserted into the accommodation space of the frame.

5. A cartridge assembly for blood analysis comprising:
a main cartridge comprising a main case having a sub-cartridge accommodation groove formed on one surface thereof, a plurality of reagent solution bags disposed inside the main case, a specimen supply part disposed inside the main case, and an opening/closing door which is elastically and rotatably coupled to another surface facing the one surface;
a sub-cartridge which is inserted into the sub-cartridge accommodation groove so as to be detachable from the main cartridge and comprises a sub-case and a plurality of quality control solution bags disposed inside the sub-case; and
a sensor card detachably coupled to the main cartridge.

6. The cartridge assembly for blood analysis of claim 5, wherein the specimen supply part comprises a first rotating body, a guide having one end part coupled to the first rotating body, a second rotating body coupled to the guide and configured to slide in a slit formed in the guide, and a needle coupled to the second rotating body, and
the opening/closing door is opened by being pressed by the second rotating body.

7. The cartridge assembly for blood analysis of claim 5, wherein the main cartridge further comprises a handle having one end part rotatably coupled to the main case.

8. The cartridge assembly for blood analysis of claim 7, wherein the handle is elastically rotated with respect to the main case.

9. The cartridge assembly for blood analysis of claim 7, wherein a handle accommodation groove is formed on an upper surface of the main case connected to the one surface of the main case, and
the handle is rotated and accommodated in the handle accommodation groove.

10. The cartridge assembly for blood analysis of claim 5, wherein the main cartridge further comprises a solution distribution part disposed on the one surface of the main case, and
the solution distribution part has a plurality of inlets connected to two or more of the reagent solution bags and the plurality of quality control solution bags, and a single outlet.

11. The cartridge assembly for blood analysis of claim 10, wherein the plurality of inlets and the outlet of the solution distribution part are spaced apart from each other in one direction.

12. The cartridge assembly for blood analysis of claim 10, wherein the sub-cartridge further comprises an accommodation connector formed with a plurality of accommodation holes connected to the plurality of the quality control solution bags, respectively, and disposed in one region of the sub-case, and
the main cartridge further comprises an insert connector formed with a plurality of protrusion parts configured to connect the solution distribution part with the plurality of quality control solution bags, respectively, by being inserted into the plurality of accommodation holes, and disposed in one region of the sub-cartridge accommodation groove corresponding to the accommodation connector.

13. The cartridge assembly for blood analysis of claim 10, wherein the main cartridge further comprises a sensor card accommodation connector disposed in the main case and connected to the outlet of the solution distribution part, and
the sensor card is at least partially inserted into the sensor card accommodation connector and connected to the outlet of the solution distribution part.

14. The cartridge assembly for blood analysis of claim 5, wherein a valve coupling hole is formed on the one surface of the main case, the valve coupling hole into which a valve of the reagent solution bag is inserted and coupled,
the main cartridge further comprises a leakage receiving part formed on a lower side of the valve coupling hole in the one surface of the main case, and
the leakage receiving part comprises a support plate protruding from the one surface of the main case toward an outer side of the main case, and a partition wall extending upward from one end part of the support plate.

15. The cartridge assembly for blood analysis of claim 14, wherein a leakage movement hole is formed between the valve coupling hole and the support plate of the leakage receiving part in the one surface of the main case, and
a level of an inner bottom surface of the main case becomes lower from the one surface side of the main case toward another surface side of the main case, which is an opposite surface of the one surface of the main case.

16. The cartridge assembly for blood analysis of claim 15, wherein a level of a bottom surface of the support plate becomes lower from the one end part side of the support plate toward a region of the support plate in contact with the one surface of the main case.
